# EUROPÄISCHE PATENTSCHRIFT

(11) **EP 1 628 611 B1**
(45) Veröffentlichungstag und Bekanntmachung des Hinweises auf die Patenterteilung: **03.10.2007**
(21) Anmeldenummer: 04732280.5
(22) Anmeldetag: 12.05.2004
(51) Int. Cl.: A61F 13/494, A61F 13/475, A61F 13/532, A61F 13/535

(54) **HYGIENEARTIKEL ZUM EINMALIGEN GEBRAUCH**
SINGLE-USE HYGIENE ARTICLE
ARTICLE HYGIENIQUE A USAGE UNIQUE

(30) Priorität: 02.06.2003 DE 10326022
(43) Veröffentlichungstag der Anmeldung: 01.03.2006
(73) Patentinhaber: Paul Hartmann Aktiengesellschaft, 89522 Heidenheim (DE)
(72) Erfinder: RÖHRL, Wolfgang, 89542 Herbrechtingen (DE); STUPPERICH, Hans-Peter, 89552 Heidenheim (DE)
(74) Vertreter: Friz, Oliver
(86) Internationale Anmeldenummer: PCT/EP2004/005053
(87) Internationale Veröffentlichungsnummer: WO 2004/105668

(56) Entgegenhaltungen:
- WO-A-01/05440
- US-A- 4 410 324
- US-A- 4 413 996
- US-A- 5 462 541
- US-A- 5 624 423
- US-A- 5 843 067

## Beschreibung

Die Erfindung betrifft einen Hygieneartikel zum einmaligen Gebrauch mit einer der dauerhaften Speicherung von Körperflüssigkeiten dienenden Saugkörperschicht, die auch superabsorbierende Materialien enthalten kann, und mit eine seitliche Auslaufsperre bildenden, im wesentlichen in einer Längsrichtung verlaufenden zumindest bereichsweise aufstehenden Cuffelementen, die zumindest entlang einer Cuffsockellinie an der körperzugewandten Seite des Artikels festgelegt sind, wobei die Cuffelemente mit variierendem Abstand der Cuffsockellinien voneinander geführt sind. Die Erfindung betrifft also insbesondere Windeln oder Windelhosen und Inkontinenzvorlagen oder Inkontinenzwindeln oder -hosen. Ein derartiger Hygieneartikel ist beispielsweise in EP 0 751 756 B1 beschrieben. Bei diesem Hygieneartikel folgen die Cuffelemente bzw. deren Cuffsockellinie dem sanduhrförmigen Saugkörper.

EP 0 254 476 B1 offenbart einen Saugkörperaufbau, wobei eine zentrale Flüssigkeitsaufnahmezone des Saugkörpers geringerer Dichte und geringeren Flächengewichts vorgesehen ist, die zumindest teilweise von einer Speicherzone höherer Dichte und höheren Flächengewichts umgeben ist. Hierdurch soll eine verbesserte Flüssigkeitsaufnahme und -verteilung, insbesondere bei schwallartiger Flüssigkeitsbeaufschlagung, erreicht werden. Ähnliches offenbart und lehrt EP 1 006 970 B1.

US-A-5,624,423 zeigt einen absorbierenden Artikel in Form einer Damenbinde mit einem seitlichen Barrieremittel in Form eines aus Schaum gebildeten Rands, der seitlichen Flüssigkeitsaustritt verhindern soll und dichtend gegen den Körper der Benutzerin angelegt sein soll. Dieser Schaumrand kann vor der Benutzung komprimiert sein und bei Einnässung expandieren.

US-A-5,462,541 zeigt einen absorbierenden Hygieneartikel in Form einer Windel mit einem ein Fenster bildenden Topsheet, wobei die das Fenster bildenden Ränder des Topsheets elastifiziert sind und aufstehende Cuffelemente bilden. Da die Cuffelemente von den Rändern des Topsheets selbst gebildet sind, ist der Verlauf einer Cuffsockellinie nicht ersichtlich.

Hiervon ausgehend liegt der vorliegenden Erfindung die Aufgabe zugrunde, einen Hygieneartikel zu schaffen, der besonders geeignet ist, bei insbesondere schwallartiger Flüssigkeitsbeaufschlagung seitliche Leckagen zu verhindern und sicherzustellen, dass die auftreffende Flüssigkeit rasch von der absorbierenden Struktur aufgenommen und dauerhaft in einer Saugkörperschicht gespeichert wird.

Diese Aufgabe wird bei einem Hygieneartikel der eingangs genannten Art erfindungsgemäß durch die Merkmale des Anspruches 1 gelöst.

Es wurde mit der Erfindung festgestellt, dass zwar die Cuffelemente mit variierendem Abstand ihrer Cuffsockellinien voneinander geführt werden können, so dass sie beispielsweise in einem Vorderbereich oder in einem Rückenbereich, also üblicherweise außerhalb eines Schrittbereichs des Hygieneartikels, weiter voneinander beabstandet sind und dass dies dann zu einer größeren Oberfläche führt, die zur Aufnahme von Flüssigkeit zur Verfügung steht. Wenn sich nämlich bei schwallartiger Flüssigkeitsbeaufschlagung die Flüssigkeit auf der Oberfläche des Hygieneartikels, also zwischen den aufstehenden Cuffelementen, verteilt, so erweist es sich für eine rasche Flüssigkeitsaufnahme als vorteilhaft, wenn die aufnehmende Fläche so groß wie möglich gewählt wird. Dies bedeutet aber, dass die Cuffelemente und die von ihnen gebildeten Taschen zur Aufnahme von Körperausscheidungen in die Nähe des Saugkörperrands gelangen, wo möglicherweise nicht ausreichend Absorptionskapazität zur Verfügung steht oder aufgrund der Wechselwirkung mit dem Rand des Saugkörpers eine den Anforderungen genügende Abdichtung oder Aufnahmekapazität nicht optimal gewährleistet ist. Hinzu kommt, dass die Anbindung der Cuffelemente an die Materialien des Hygieneartikels die Gefahr kapillaren Flüssigkeitstransports zu den Rändern des Hygieneartikels hin in ungewollter Weise begünstigt, sofern dort keine Absorptionskapazität zur Verfügung steht. Mit der vorliegenden Erfindung wurde nun erkannt, dass durch die Erhöhung des Flächengewichts eines absorbierenden Materials der Saugkörperschicht gerade dort, wo der Abstand der Cuffsockellinien voneinander größer ist als anderen Orts, eine bessere und weniger störanfällige Flüssigkeitsaufnahmecharakteristik des Hygieneartikels im Gebrauch erreicht werden kann. Diese Erhöhung des Flächengewichts kann zum einen in Querrichtung zu den Seitenrändern hin vorgesehen werden, so dass in Querrichtung beidseits außerhalb eines zentralen Bereichs beidseits Bereiche höheren Flächengewichts als in dem zentralen Bereich vorgesehen sind. Die Zunahme des Flächengewichts kann dabei kontinuierlich oder diskontinuierlich sein.

Es ist aber auch denkbar, dass in Längsrichtung zu wenigstens einem Längsende des Hygieneartikels hin ein zunehmendes Flächengewicht vorgesehen wird. Dies führt dann im Vorderbereich und/oder im Rückenbereich zu einem höheren Flächengewicht als in einem mittleren Bereich, wobei in dem Bereich höheren Flächengewichts dann erfindungsgemäß zumindest in einem Teilbereich der Abstand der Cuffsockellinien voneinander größer ist als außerhalb dieses Teilbereichs.

Mit der Erfindung wurde also erkannt, dass die Führung der Cuffelemente und deren Cuffsockellinien dann in vorteilhafter Weise zur Schaffung einer großen Flüssigkeitsaufnahmefläche herangezogen werden können, wenn gerade im Bereich größeren Abstands der Cuffsockellinien voneinander eine Erhöhung der Flüssigkeitsaufnahmekapazität zumindest der zur dauerhaften Speicherung von Körperflüssigkeiten dienenden Saugkörperschicht vorgesehen wird, und zwar zumindest im Bereich der Cuffsockellinien.

Obschon die erfindungsgemäße Erkenntnis auch in einem mittleren Schrittbereich des Hygieneartikels Anwendung finden kann, erweist es sich als besonders vorteilhaft, wenn der erwähnte Teilbereich außerhalb eines mittleren Längsabschnitts in einem Vorderbereich und/oder in einem Rückenbereich des Hygieneartikels angeordnet ist, da dort der Hygieneartikel und seine Saugkörperstruktur in Querrichtung unproblematischer ausladend konfiguriert werden kann, als diens im Schrittbereich möglich ist. Da die Funktionalität der Hygieneartikel gerade bei liegenden oder schlafenden Benutzern sichergestellt werden soll, kommt dem Vorderbereich und dem Rückenbereich eine größere Bedeutung bei der Flüssigkeitsabsorption zu, als dies bislang vermutet wurde. Zwar wurden bereits "boy"-Windeln vorgeschlagen mit erhöhter Saugkapazität in einem vorderen Bereich. Diese Saugkapazität war dann aber zentral, also mittig, angeordnet. Nach der vorliegenden Erfindung und der ihr zugrunde liegenden Erkenntnis soll aber eine höhere Saugkapazität durch ein höheres Flächengewicht der absorbierenden Materialien, zumindest der zur dauerhaften Speicherung von Körperflüssigkeiten dienenden Saugkörperschicht, im Bereich maximalen Abstands der Cuffsockellinien bei den Cuffsockellinien bereitgestellt werden.

Nach einer weiteren bevorzugten Ausführungsform der Erfindung wird vorgeschlagen, dass die Cuffelemente so geführt sind, dass der Abstand der Cuffsockellinien voneinander in Längsrichtung wenigstens ein Maximum, vorzugsweise je ein Maximum im Vorderbereich und im Rückenbereich des Hygieneartikels, durchläuft. Wenn davon die Rede ist, dass ein Maximum durchlaufen wird, so ist dies dahingehend zu verstehen, dass der Abstand der Cuffsockellinien in einer Längsrichtung zunimmt, ein Maximum erreicht und dann wieder abnimmt, sich die Cuffsockellinien also wieder einander nähern.

Die Cuffelemente selbst umfassen dem Fachmann zur Ausbildung von seitlichen Auslaufsperren an sich bekannte Materialien, wie Vliesstoffe, zum Beispiel Karden- Spinn- oder Meltblownvliesstoffe, insbesondere Vliesstofflaminate, welche Spinn (S)- und Meltlown (M) - Vliesstofflagen umfassen, beispielsweise SM-, SMS-, SMMS- Vliesstoffe. Vorzugsweise kommen hydrophobe Materialien zum Einsatz. In Frage kommen außerdem Cuffelemente aus Folien- oder Schaumstoffmaterialien.

Für die Ausbildung der Saugkörperschicht, deren Flächengewicht in Querrichtung und/oder in Längsrichtung zunimmt, bestehen an sich keine besonderen, von üblichen Saugkörperschichten zur dauerhaften Speicherung von Körperflüssigkeiten abweichenden Anforderungen. Es könnte sich hierbei um eine Schicht reinen Fasermaterials, insbesondere gefluffter Zellstofffasern, handeln, die aufgrund ihrer Hydrophilität für die dauerhafte Speicherung von Flüssigkeit geeignet sind. In Frage kommen außerdem synthetische, insbesondere thermoplastische Fasern, ggf. in Mischung mit Zellstoffasern. Es erweist sich als besonders vorteilhaft, wenn die Saugkörperschicht superabsorbierende Materialien, insbesondere partikelförmige superabsorbierende Materialien umfasst. Vorzugsweise umfasst die Saugkörperschicht eine homogene Mischung aus Fasern und partikelförmigen superabsorbierenden Materialien. Die Saugkörperschicht kann weiterhin Schaumstoffmaterialien umfassen oder daraus bestehen.

Nach einer Ausführungsform der Erfindung wird vorgeschlagen, dass die Sockellinien der beiderseits verlaufenden Cuffelemente im Vorderbereich und/oder im Rückenbereich einander treffen. Solchenfalls wäre eine in Längsrichtung abgedichtete Tasche zur Aufnahme von Körperausscheidungen geschaffen. Die Cuffelemente selbst wären dann vorzugsweise so ausgebildet, dass sich ihre distalen Enden ebenfalls berühren oder überlappen. Es wäre dann eine Art fensterartige Öffnung geschaffen.

Alternativ hierzu wäre es aber denkbar und insbesondere im Hinblick auf fertigungstechnische Prozesse vorteilhaft, wenn die Sockellinien der beidseits verlaufenden Cuffelemente im Vorderbereich und/oder im Rückenbereich voneinander beabstandet über das Längsende des Hygieneartikels auslaufen.

Die Ansprüche 9 bis 11 betreffen bevorzugte Abmessungen bei der Führung der aufstehenden Cuffelemente nach der Erfindung.

In weiterer Präzisierung des Erfindungsgedankens wird vorgeschlagen, den Hygieneartikel so auszubilden, dass das Flächengewicht der besagten Saugkörperschicht oder eines ihrer absorbierenden Materialien in Querrichtung oder Längsrichtung um 30 bis 200 %, insbesondere um 30 bis 150 %, und vorzugsweise um 50 bis 120 %, zunimmt.

Nach einer bevorzugten Ausführungsform der Erfindung beträgt das Flächengewicht eines absorbierenden Materials der Saugkörperschicht außerhalb des besagten Teilbereichs 200 bis 500 g/m², insbesondere 250 bis 450 g/m², und vorzugsweise 280 bis 350 g/m². Es beträgt innerhalb des besagten Teilbereichs 250 bis 1000 g/m², insbesondere 350 bis 750 g/m², und bevorzugtermaßen 500 bis 700 g/m². Das absorbierende Material der Saugkörperschicht kann durch die Faserkomponente, insbesondere gefluffte Cellulosefasern, gebildet sein.

Es wird darauf hingewiesen, dass oberhalb und/oder unterhalb der betrachteten Saugkörperschicht weitere insbesondere der Flüssigkeitsaufnahme oder -speicherung dienende Saugkörperkomponenten vorgesehen sein können. So kann eine vorzugsweise offenporige Flüssigkeitsaufnahmeschicht vorgesehen werden , die aber vorzugsweise nicht der dauerhaften Speicherung, sondern lediglich der Flüssigkeitsaufnahme, -zwischenspeicherung und -verteilung dient. Es kann sich außerdem als vorteilhaft erweisen, dass unterhalb der betrachteten Saugkörperschicht eine weitere, vorzugsweise SAP-freie Schicht, vorgesehen werden kann, die als Unterlage dient und insbesondere das Austreten körnigen superabsorbierenden Partikelmaterials aus der eigentlichen Saugkörperschicht verhindern soll.

Nach einer besonders bevorzugten Ausführungsform der vorliegenden Erfindung sind die Cuffelemente durch in Längsrichtung verlaufende Bahnen oder Bahnabschnitte ausgebildet. Wenn dann die Anfügung dieser Bahnabschnitte durch entsprechende Führung des Verlaufs der jeweiligen Cuffsockellinie festgelegt wird, so sind die Cuffelemente so ausgebildet, dass der Abstand eines distalen Endes der aufstehenden Cuffelemente von der jeweiligen Cuffsockellinie umso größer wird, je größer der Abstand der Cuffsockellinien voneinander ist. Dies ist auch zweckmäßig, da hierdurch in einem breit ausladenden Aufnahmebereich die aufstehenden Cuffelemente dann eine größere effektive Höhe einnehmen können. In weiterer Ausbildung dieses Erfindungsgedankens erweist es sich als vorteilhaft, wenn der Abstand eines distalen Endes der aufstehenden Cuffelemente von der Cuffsockellinie innerhalb des besagten Teilbereichs höheren Flächengewichts größer ist als außerhalb dieses Teilbereichs.

Es hat sich als zweckmäßig und vorteilhaft erwiesen, wenn der Abstand eines distalen Endes der aufstehenden Cuffelemente von der Cuffsockellinie innerhalb des besagten Teilbereichs 40 bis 60 mm beträgt. Der Abstand wird hierbei als effektive Länge bei flachgelegtem Cuffelement verstanden.

Außerhalb des besagten Teilbereichs beträgt der Abstand vorzugsweise 30 bis 40 mm.

Weitere Merkmale, Einzelheiten und Vorteile der Erfindung ergeben sich aus der zeichnerischen Darstellung und nachfolgenden Beschreibung bevorzugter Ausführungsformen der Erfindung. In der Zeichnung zeigt:
- Figur 1: eine Draufsicht auf eine bevorzugte Ausführungsform eines erfindungsgemäßen Hygieneartikels;
- Figur 2: eine Schnittansicht des Hygieneartikels nach Figur 1 mit Schnittebene II-II in Figur 1;
- Figur 3: eine Draufsicht auf einen Saugkörper einer weiteren Ausführungsform mit angedeutetem Verlauf der Cuffsockellinien;
- Figur 4: eine Draufsicht auf einen Saugkörper einer weiteren Ausführungsform mit angedeutetem Verlauf der Cuffsockellinien; und
- Figur 5: eine Längsseitenansicht des Saugkörpers nach Figur 3.

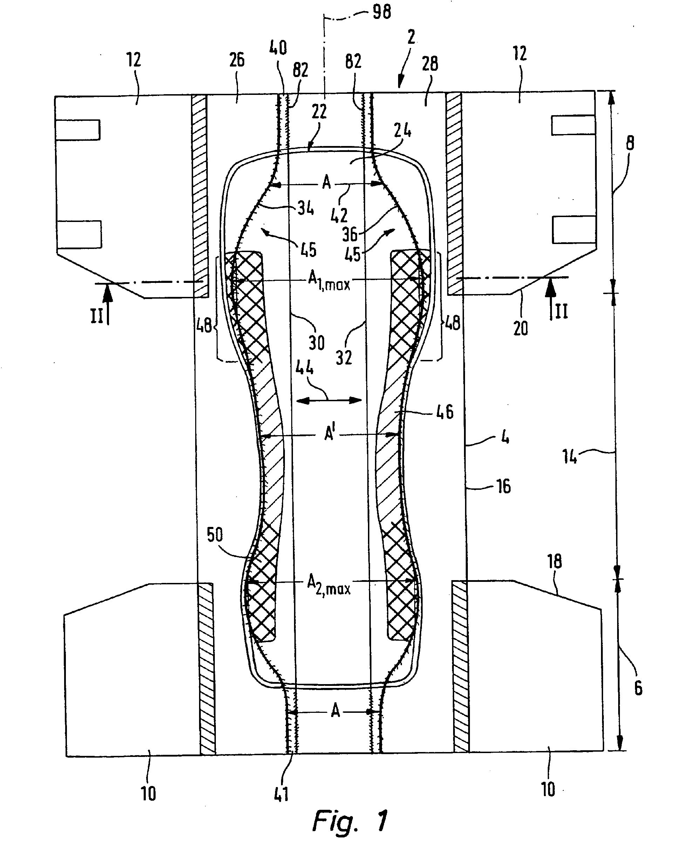

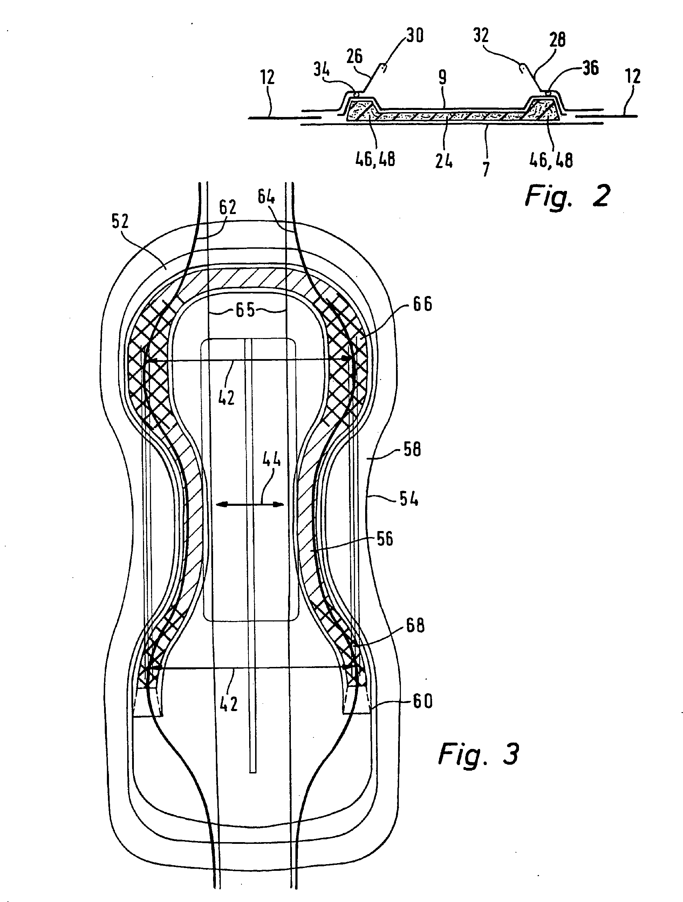

Figur 1 zeigt eine Draufsicht auf die körperzugewandte Seite eines insgesamt mit dem Bezugszeichen 2 bezeichneten Hygieneartikels in Form einer Inkontinenzwindel im flachgelegten Zustand. Figur 2 zeigt eine entsprechende Schnittansicht mit Schnittebene entlang der Linie II-II. Der Hygieneartikel 2 umfasst ein Windelchassis 4 mit in einem Vorderbereich 6 und einem Rückenbereich 8 beidseits direkt oder indirekt angefügten Seitenklappen 10 bzw. 12. Der Bereich zwischen Vorderbereich 6 und Rückenbereich 8 sei als Schrittbereich 14 bezeichnet. Ein Seitenlängsrand 16 des Windelchassis 4 im Schrittbereich 14 begrenzt zusammen mit den daran anschließenden Flanken 18, 20 der Seitenklappen 10, 12 die Beinöffnungen des Benutzers im angelegten Zustand des Hygieneartikels.

Während das Chassis auf der körperabgewandten Seite eine flüssigkeitsundurchlässige Lage 7 aufweist, sind die Seitenklappen 10, 12 aus einem atmungsaktiven insbesondere luftdurchlässigen Vliesmaterial gebildet.

Der Hygieneartikel 2 umfasst des Weiteren einen Saugkörper 22 mit einer nicht dargestellten offenporigen Flüssigkeitsaufnahme- und -verteilerschicht, die den Saugkörper nur teilweise überfängt. Des Weiteren umfasst der Saugkörper 22 eine der dauerhaften Speicherung von Körperflüssigkeiten dienende Saugkörperschicht 24, die nachfolgend noch näher beschrieben werden wird. Die Saugkörperschicht 24 liegt auf einer in den Figuren 1 und 2 nicht dargestellten dünnen Lage aus gefluffter Zellulose, die ein gleichförmiges Flächengewicht von etwa 140 g/m² aufweist.

Mit Bezugszeichen 26, 28 sind in Längsrichtung verlaufende zumindest im Schrittbereich aufstehende Cuffelemente bezeichnet, deren freie distale Ränder 30, 32 vorzugsweise mit einem Elastifizierungsmittel versehen sind. Um ein Nachaußenklappen der Cuffelemente zu verhindern sind die distalen Ränder 30, 32 im Rücken- und Vorderbereich nach innen an die Oberseite des Hygieneartikels mittels üblicher Verfahren wie Verklebung oder Verschweißung angefügt. Die Cuffelemente 26, 28 sind außerdem entlang einer jeweiligen Cuffsockellinie 34, 36 an die Oberseite des Hygieneartikels 2 angefügt, insbesondere mit einer zumindest das Chassis 4 im wesentlichen überfangenden Topsheetlage 9 (Figur 2) verbunden.

Die Cuffsockellinien 34, 36 erstrecken sich wie die Cuffelemente zwar grundsätzlich in Längsrichtung 38 des Hygieneartikels, sie verlaufen jedoch nicht parallel zur Längsrichtung 38, sondern sind mit variierendem Abstand der Cuffsockellinien 34, 36 voneinander geführt.

Ausgehend von einem Längsende 40 des Hygieneartikels 2 verlaufen die Cuffsockellinien 34, 36 zunächst exakt parallel zur Längsrichtung 38 mit einem Abstand 42, der durch den Wert A bezeichnet ist. Noch innerhalb des Rückenbereichs 8 verlaufen die Cuffsockellinien 34, 36 dann stetig bogenförmig nach außen, so dass sich ihr Abstand 42 stetig vergrößert, bis zu einem maximalen Wert A₁ₘₐₓ. Der Abstand fällt dann von A_{1,max} wiederum stetig auf einen Wert A' im Schrittbereich 14 des Hygieneartikels 2 ab, der jedoch einige Millimeter größer ist als der Abstand A. Im weiteren Verlauf der Cuffsockellinien 34, 36 vom Schrittbereich in den Vorderbereich 6 vergrößert sich der Abstand der Cuffsockellinien 34, 36 wiederum stetig auf einen Wert A₂,ₘₐₓ, der etwas geringer ist als der Wert A_{1,max}, um dann zum anderen Längsende 44 hin wieder auf den Ausgangswert A abzufallen.

Die Cuffelemente 26, 28 sind also so geführt, dass der Abstand der Cuffsockellinien 34, 36 voneinander in Längsrichtung zwei Maxima durchläuft, die vorzugsweise zumindest teilweise im Vorderbereich 6 oder im Rückenbereich 8 des Hygieneartikels angeordnet sind. Wenn wie im dargestellten Fall außerdem die distalen Ränder 30, 32 der Cuffelemente im Rücken- und Vorderbereich nach innen an die Oberseite des Hygieneartikels angefügt sind (Cuffendenfixierung 82), resultieren taschenförmige Barrieren (45), die neben der Primärfunktion der Cuffelemente, nämlich seitlichen Auslaufschutz zu bieten, eine Auslaufbarriere auch hin zu den Taillenrändern bilden. Vorteilhaft ist insbesondere, wenn wie dargestellt die Cuffsockellinie im Vorder- und/oder Rückenbereich im Wesentlichen gleichsam in den Bereich der Cuffendenfixierung hineinläuft

Die zur dauerhaften Speicherung von Körperflüssigkeiten dienende Saugkörperschicht 24 selbst ist strukturiert ausgebildet, sie weist also kein über ihre Erstreckung gleichförmiges Flächengewicht auf, sondern ihr Flächengewicht nimmt in Querrichtung 44 zu den Seitenrändern 16 hin zu, indem sie dort einen Bereich 46 mit höherem Gewicht des absorbierenden Saugkörpermaterials, das durch eine homogene Mischung von Zellstofffasern und superabsorbierendem partikelförmigem Material gebildet ist, aufweist. Dies ist auch aus der Schnittansicht der Figur 2 ersichtlich. Dieser Bereich 46 erhöhten Flächengewichts erstreckt sich entlang eines Randbereichs des Saugkörpers (schraffiert angedeutet) und umfasst zumindest einen Teilbereich 48 (kreuzschraffiert angedeutet), in dem der Abstand der Cuffsockellinien 34, 36 voneinander größer ist als außerhalb dieses Teilbereichs 48. Der größte Abstand 42 der Cuffsockellinien 34, 36 oder ein Maximum des Abstands 42 der Cuffsockellinien 34, 36 voneinander ist in einem Längsabschnitt des Hygieneartikels angeordnet, wo die Saugkörperschicht 24 ein erhöhtes Flächengewicht aufweist, wo also auch der Bereich 46 vorgesehen ist. Durch die Verstärkung der Saugkörperschicht 24 im Randbereich ist dort eine erhöhte Speicherkapazität erreicht. Dies wirkt sich in den Teilbereichen 48, 50 des Bereichs 46, in denen der Abstand 42 der Cuffsockellinien 34, 36 voneinander größer ist, besonders vorteilhaft aus. Es führt zu einer verbesserten Absorptionscharakteristik, also insbesondere zu rascherer Flüssigkeitsaufnahme. Es wird im Vorderbereich 6 und im Rückenbereich 8 durch den dort zunehmenden Abstand der Cuffsockellinien 34, 36 voneinander eine große beaufschlagbare Flüssigkeitsaufnahmefläche geschaffen, die sich insbesondere bei Stuhlinkontinenz im hinteren Produktbereich vorteilhaft auswirkt. Es sind breit ausladende Aufnahmeräume für Körperausscheidungen geschaffen, wobei gerade im Bereich der Flüssigkeitsbarrieren durch die aufstehenden Cuffelemente 30, 32 eine erhöhte Speicherkapazität und damit auch ein erhöhter Schutz gegen seitliche Leckagen erreicht ist.

Figur 3 zeigt die Draufsicht auf einen Saugkörper 52 einer weiteren Ausführungsform des erfindungsgemäßen Hygieneartikels. Wie der Saugkörper 24 des Hygieneartikels nach Figur 1 ist der Saugkörper 52 in Querrichtung 44 in Richtung auf die seitlichen Längsränder 54 mit einem zunehmenden Flächengewicht in einem schraffiert angedeuteten Bereich 56 ausgebildet. Man erkennt eine dünne mit gleichförmigem Flächengewicht ausgebildete Fluffschicht 58, die zwischen der Saugkörperschicht 60 und einem nicht dargestellten Backsheet angeordnet ist. Die Cuffelemente sind durch ihre Cuffsockellinien 62, 64 und ihre distalen Enden 65 angedeutet. Sie sind wieder mit variierendem Abstand A der Cuffsockellinien 62, 64 in Längsrichtung geführt. Und wiederum ist zumindest in einem Teilbereich 66, 68 des Bereichs 56 höheren Flächengewichts der Cuffsockelabstand 42 größer als außerhalb dieses Teilbereichs 66, 68.

Im Unterschied zu Figur 1 ist der Bereich 56 höheren Flächengewichts im Vorderbereich des Saugkörpers 52 ringartig geschlossen, so wie dies aus Figur 3 ersichtlich ist. Diese Ausführungsform bietet besondere Vorteile im Fall der Verwendung innerhalb eines Inkontinenzartikel, der bevorzugt für Männer Verwendung findet.

Es wird darauf hingewiesen, dass es sich bei beiden Ausführungsformen als vorteilhaft erweist, wenn mit dem zunehmenden Flächengewicht auch eine zunehmende Dicke der Saugkörperschicht und damit des Saugkörpers einhergeht. Dies unterstützt die Leckagesicherheit im Bereich der Saugkörperränder ganz wesentlich.

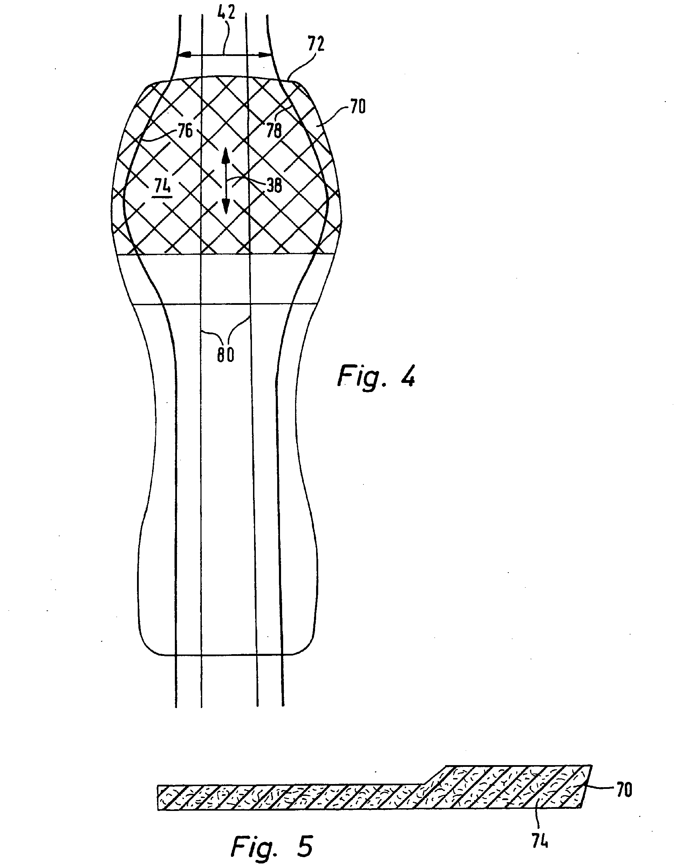

Die Figuren 4 und 5 zeigen eine Draufsicht sowie eine Schnittansicht durch eine weitere nach der Erfindung ausgebildete Saugkörperschicht 70. Im Unterschied zu den Ausführungsformen nach Figuren 1 und 2 nimmt das Flächengewicht der Saugkörperschicht 70 in Längsrichtung 38 in Richtung auf ein Längsende 72 des in Figur 5 nicht dargestellten Hygieneartikels im Ganzen zu. Die Dickenzunahme der Saugkörperschicht 70 erfolgt über einen rampenförmigen Anstieg zwischen Schrittbereich und Vorderbereich oder Rückenbereich des herzustellenden Hygieneartikels. Man erkennt den Bereich 74 erhöhten Flächengewichts. Weiter ist schematisch dargestellt der Verlauf von Cuffsockellinien 76, 78 und der distalen Enden 80 der Cuffelemente. Man erkennt, das die Cuffsockellinien 76, 78 ein Maximum des Abstands voneinander durchlaufen, der im Bereich 74 erhöhten Flächengewichts zu liegen kommt.

## Patentansprüche

1. Hygieneartikel (2) zum einmaligen Gebrauch mit einer der dauerhaften Speicherung von Körperflüssigkeiten dienenden Saugkörperschicht (24, 60, 70), die auch superabsorbierende Materialien enthalten kann, und mit eine seitliche Auslaufsperre bildenden im wesentlichen in einer Längsrichtung verlaufenden zumindest bereichsweise aufstehenden Cuffelementen (26, 28), die zumindest entlang einer Cuffsockellinie (34, 36; 62, 64; 76, 78) an der körperzugewandten Seite des Artikels festgelegt sind, wobei die Cuffelemente (26, 28) mit variierendem Abstand der Cuffsockellinien (34, 36; 62, 64; 76, 78) voneinander geführt sind, **dadurch gekennzeichnet, dass** die Saugkörperschicht (24, 60, 70) in Querrichtung (44) des Hygieneartikels zu den Seitenrändern (16) hin und/oder in Längsrichtung (38) zu wenigstens einem Längsende (40) hin ein zunehmendes Flächengewicht eines absorbierenden Materials dieser Schicht aufweist und dass der Bereich (46, 56, 74) größeren Flächengewichts zumindest einen Teilbereich (48, 50, 66, 68) aufweist, in dem der Abstand (42) der Cuffsockellinien (34, 36; 62, 64; 76, 78) voneinander größer ist als außerhalb dieses Teilbereichs (48, 50, 66, 68) und dass der Abstand eines distalen Endes (30, 32) der aufstehenden Cuffelemente (26, 28) von der Cuffsockellinie (34, 36; 62, 64; 76, 78) innerhalb des besagten Teilbereichs (48, 50, 66, 68) größer ist als außerhalb dieses Teilbereichs (48, 50, 66, 68).

2. Hygieneartikel nach Anspruch 1, **dadurch gekennzeichnet, dass** dieser Teilbereich (48, 50, 66, 68) außerhalb eines mittleren Längsabschnitts in einem Vorderbereich (6) und/oder Rückenbereich (8) des Hygieneartikels angeordnet ist.

3. Hygieneartikel nach Anspruch 1 oder 2, **dadurch gekennzeichnet, dass** die Cuffelemente (26, 28) so geführt sind, dass der Abstand (42) der Cuffsockellinien (34, 36; 62, 64; 76, 78) voneinander in Längsrichtung (38) zumindest ein Maximum durchläuft.

4. Hygieneartikel nach Anspruch 3, **dadurch gekennzeichnet, dass** das Maximum vollständig innerhalb dieses Teilbereichs (48, 50, 66, 68) des Bereichs (46, 56, 74) größeren Flächengewichts liegt.

5. Hygieneartikel nach Anspruch 3 oder 4, **dadurch gekennzeichnet, dass** das Maximum des Abstands der Cuffsockellinien (34, 36; 62, 64; 76, 78) voneinander in einem Vorderbereich (6) und/oder in einem Rückenbereich (8) des Hygieneartikels angeordnet ist.

6. Hygieneartikel nach einem oder mehreren der vorstehenden Ansprüche, **dadurch gekennzeichnet, dass** die Saugkörperschicht (24, 60, 70) eine Mischung aus Fasern und partikelförmigen superabsorbierenden Materialien umfasst.

7. Hygieneartikel nach einem oder mehreren der vorstehenden Ansprüche, **dadurch gekennzeichnet, dass** die Cuffsockellinien (34, 36; 62, 64; 76, 78) der beidseits verlaufenden Cuffelemente (26, 28) im Vorderbereich (6) und/oder im Rückenbereich (8) einander treffen.

8. Hygieneartikel nach einem oder mehreren der vorstehenden Ansprüche, **dadurch gekennzeichnet, dass** die Cuffsockellinien (34, 36; 62, 64; 76, 78) der beidseits verlaufenden Cuffelemente (26, 28) im Vorderbereich (6) und/oder im Rückenbereich (8) voneinander beabstandet über das Längsende (40) auslaufen.

9. Hygieneartikel nach einem oder mehreren der vorstehenden Ansprüche, **dadurch gekennzeichnet, dass** der Abstand (42) der Cuffsockellinien (34, 36; 62, 64; 76, 78) voneinander in einem mittleren Längsabschnitt des Hygieneartikels und außerhalb des besagten Teilbereichs (48, 50, 66, 68) 130 - 170 mm, insbesondere 140 - 165 mm beträgt.

10. Hygieneartikel nach einem oder mehreren der vorstehenden Ansprüche, **dadurch gekennzeichnet, dass** der Abstand (42) der Cuffsockellinien (34, 36; 62, 64; 76, 78) voneinander in dem besagten Teilbereich (48, 50, 66, 68) 160 - 220 mm, insbesondere 180 - 200 mm, insbesondere 185 - 195 mm beträgt.

11. Hygieneartikel nach einem oder mehreren der vorstehenden Ansprüche, **dadurch gekennzeichnet, dass** der Abstand (42) der Cuffsockellinien (34, 36; 62, 64; 76, 78) voneinander in einem ein Längsende des Hygieneartikels einschließenden Produktendbereich 100 - 180 mm, insbesondere 100 - 160 mm, insbesondere 100 - 150 mm, insbesondere 100 - 140 mm, insbesondere 100 - 130 mm, insbesondere 105 - 120 mm beträgt.

12. Hygieneartikel nach einem oder mehreren der vorstehenden Ansprüche, **dadurch gekennzeichnet, dass** das Flächengewicht der Saugkörperschicht (24, 60, 70) oder eines ihrer absorbierenden Materialien in Querrichtung (44) oder Längsrichtung (38) um 30 - 200 %, insbesondere um 30 - 150 %, insbesondere 50 - 120 %, zunimmt.

13. Hygieneartikel nach einem oder mehreren der vorstehenden Ansprüche, **dadurch gekennzeichnet, dass** das Flächengewicht eines absorbierenden Materials der Saugkörperschicht (24, 60, 70) außerhalb des besagten Teilbereichs (48, 50, 66, 68) 200 - 500 g/m², insbesondere 250 - 450 g/m², insbesondere 280 - 350 g/m² beträgt.

14. Hygieneartikel nach einem oder mehreren der vorstehenden Ansprüche, **dadurch gekennzeichnet, dass** das Flächengewicht eines absorbierenden Materials der Saugkörperschicht (24, 60, 70) innerhalb des besagten Teilbereichs (48, 50, 66, 68) 250 - 1000 g/m², insbesondere 350 - 750 g/m², insbesondere 500 - 700 g/m² beträgt.

15. Hygieneartikel nach einem oder mehreren der vorstehenden Ansprüche, **dadurch gekennzeichnet, dass** der Abstand eines distalen Endes der aufstehenden Cuffelemente (26, 28) von der Cuffsockellinie (34, 36; 62, 64; 76, 78) innerhalb des besagten Teilbereichs (48, 50, 66, 68) 40 - 60 mm beträgt.

16. Hygieneartikel nach einem der vorstehenden Ansprüche, **dadurch gekennzeichnet, dass** der Abstand eines distalen Endes der aufstehenden Cuffelemente (26, 28) von der Cuffsockellinie (34, 36; 62, 64; 76, 78) außerhalb des besagten Teilbereichs (48, 50, 66, 68) 30 - 40 mm beträgt.

## Claims

1. Hygiene article (2) for single use with an absorbent core layer (24, 60, 70) for long-term storage of body fluids which may contain superabsorbing materials, and with cuff elements (26, 28) forming sideward barrier against leakage and extending substantially in a longitudinal direction, which are at least partially elevated and which are attached to the side of the article facing the body of the user at least along a cuff base line (34, 36; 62, 64; 76, 78), wherein the cuff elements (26, 28) extend with varying distance between their cuff base lines (34, 36; 62, 64; 76, 78), **characterized in that** the absorbent core layer (24, 60, 70) has a basis weight of an absorbent material which of this layer increases in the transverse direction (44) of the hygiene article towards the side edges (16) and/or in the longitudinal direction (38) towards at least one longitudinal end (40), and that the region of increased basis weight (46, 56, 74) has at least one partial region (48, 50, 66, 68) in which the distance (42) between the cuff base lines (34, 36; 62, 64; 76, 78) is larger than outside of this partial region (48, 50, 66, 68) and that the distance of a distal end (30, 32) of the elevated cuff elements (26, 28) from the cuff base line (34, 36; 62, 64; 76, 78) within said partial region (48, 50, 66, 68) is greater than it is outside of said partial region (48, 50, 66, 68).

2. Hygiene article according to claim 1, **characterized in that** the partial region (48, 50, 66, 68) is disposed outside of a middle longitudinal section in a forward section (6) and/or in a rear area (8) of the hygiene article.

3. Hygiene article according to claim 1 or 2, **characterized in that** the cuff elements (26, 28) extend in such a fashion that the distance (42) between the cuff base lines (34, 36; 62, 64; 76, 78) along the longitudinal direction (38) has at least one maximum.

4. Hygiene article according to claim 3, **characterized in that** the maximum is completely within said partial region (48, 50, 66, 68) of the region of increased basis weight (46, 56, 74).

5. Hygiene article according to claim 3 or 4, **characterized in that** the maximum of the distance between the cuff base lines (34, 36; 62, 64; 76, 78) is in a forward region (6) and/or in a rear region (8) of the hygiene article.

6. Hygiene article according to any one or more of the preceding claims, **characterized in that** the absorbent core layer (24, 60, 70) includes a mixture of fibers and particle shaped superabsorbing materials.

7. Hygiene article according to any one or more of the preceding claims, **characterized in that** the cuff base lines (34, 36; 62, 64; 76, 78) of the cuff elements (26, 28) extending along both sides in the forward region (6) and/or in the back region (8) join onto each other.

8. Hygiene article according to any one or more of the preceding claims, **characterized in that** the cuff base lines (34, 36; 62, 64; 76, 78) of the cuff elements (26, 28) which extend along both sides extend past the longitudinal end (40) having a distance between each other in the forward region (6) and/or in the rear region (8).

9. Hygiene article according to any one or more of the preceding claims, **characterized in that** the distance (42) between the cuff base lines (34, 36; 62, 64; 76, 78) in a middle longitudinal section of the hygiene article and outside of said partial region (48, 50, 66, 68) is 130 to 170 mm, in particular 140-165 mm.

10. Hygiene article according to any one or more of the preceding claims, **characterized in that** the distance (42) between the cuff base lines (34, 36; 62, 64; 76, 78) in the said partial region (48, 50, 66, 68) is 160-220 mm, in particular 180-200 mm, in particular 185-195 mm.

11. Hygiene article according to any one or more of the preceding claims, **characterized in that** the distance (42) between the cuff base lines (34, 36; 62, 64; 76, 78) in the product end region including the longitudinal end of the hygiene article assumes values of 100-180 mm, in particular 100-160 mm, in particular 100-150 mm, in particular 100-140 mm, in particular 100-130 mm, in particular 105-120 mm.

12. Hygiene article according to any one or more of the preceding claims, **characterized in that** the basis weight of the absorbent core layer (24, 60, 70) or of one of its absorbing materials increases by 30-200 %, in particular by 30-150 %, in particular by 50-120 % in the transverse (44) or in the longitudinal direction (38).

13. Hygiene article according to any one or more of the preceding claims, **characterized in that** the surface density of an absorbent material in the absorbent core layer (24, 60, 70) outside of said partial region (48, 50, 66, 68) is 200-500 g/m², in particular 250-450 g/m², in particular 280-350 g/m².

14. Hygiene article according to any one or more of the preceding claims, **characterized in that** the basis weight of an absorbent material of the absorbent core layer (26, 60, 70) within said partial region (48, 50, 66, 68) is 250-1000 g/m², in particular 350-750 g/m², in particular 500-700 g/m².

15. Hygiene article according to any one or more of the preceding claims, **characterized in that** the distance between a distal end of the elevated cuff elements (26, 28) and the cuff base line (34, 36; 62, 64; 76, 78) within said partial region (48, 50, 66, 68) is 40-60 mm.

16. Hygiene article according to any one of the preceding claims, **characterized in that** the distance between a distal end of the elevated cuff elements (26, 28) and the cuff base lines (34, 36; 62, 64; 76, 78) outside of said partial region (48, 50, 66, 68) is 30-40 mm.

## Revendications

1. Article d'hygiène (2) à usage unique comprenant une couche à corps absorbant (24, 60, 70) servant au stockage durable de liquides corporels, laquelle couche peut également contenir des matériaux superabsorbants, et comprenant des éléments de revers (26, 28) verticaux au moins par sections s'étendant essentiellement dans un sens longitudinal et formant une barrière d'écoulement latérale, lesquels éléments de revers sont fixés sur le côté orienté vers le corps de l'article au moins le long d'une ligne de base de revers (34, 36 ; 62, 64 ; 76, 78), les éléments de revers (26, 28) étant guidés l'un par rapport à l'autre à une distance variable des lignes de base de revers (34, 36 ; 62, 64 ; 76, 78), **caractérisé en ce que** la couche à corps absorbant (24, 60, 70) présente un poids par unité de surface croissant d'un matériau absorbant de cette couche dans le sens transversal (44) de l'article d'hygiène vers les bords latéraux (16) et/ou dans le sens longitudinal (38) vers au moins une extrémité longitudinale (40), **en ce que** la zone (46, 56, 74) d'un poids par unité de surface plus important présente au moins une zone partielle (48, 50, 66, 68), dans laquelle la distance (42) qui sépare les lignes de base de revers (34, 36 ; 62, 64 ; 76, 78) les unes des autres est plus importante qu'à l'extérieur de cette zone partielle (48, 50, 66, 68) et **en ce que** la distance d'une extrémité distale (30, 32) des éléments de revers verticaux (26, 28) par rapport à la ligne de base de revers (34, 36 ; 62, 64 ; 76, 78) à l'intérieur de ladite zone partielle (48, 50, 66, 68) est plus importante qu'à l'extérieur de cette zone partielle (48, 50, 66, 68).

2. Article d'hygiène selon la revendication 1, **caractérisé en ce que** cette zone partielle (48, 50, 66, 68) est agencée en-dehors d'un segment longitudinal central dans une zone avant (6) et/ou une zone arrière (8) de l'article d'hygiène.

3. Article d'hygiène selon la revendication 1 ou 2, **caractérisé en ce que** les éléments de revers (26, 28) sont guidés de façon à ce que la distance (42) qui sépare les lignes de base de revers (34, 36 ; 62, 64 ; 76, 78) les unes des autres atteigne au moins un maximum dans le sens longitudinal (38).

4. Article d'hygiène selon la revendication 3, **caractérisé en ce que** le maximum se situe entièrement à l'intérieur de cette zone partielle (48, 50, 66, 68) de la zone (46, 56, 74) présentant un poids par unité de surface plus important.

5. Article d'hygiène selon la revendication 3 ou 4, **caractérisé en ce que** la distance maximale entre les lignes de base de revers (34, 36 ; 62, 64 ; 76, 78) se situe dans une zone avant (6) et/ou dans une zone arrière (8) de l'article d'hygiène.

6. Article d'hygiène selon une ou plusieurs des revendications précédentes, **caractérisé en ce que** la couche à corps absorbant (24, 60, 70) comprend un mélange de fibres et de matériaux superabsorbants en forme de particules.

7. Article d'hygiène selon une ou plusieurs des revendications précédentes, **caractérisé en ce que** les lignes de base de revers (34, 36 ; 62, 64 ; 76, 78) des éléments de revers (26, 28) s'étendant des deux côtés se rejoignent dans la zone avant (6) et/ou dans la zone arrière (8).

8. Article d'hygiène selon une ou plusieurs des revendications précédentes, **caractérisé en ce que** les lignes de base de revers (34, 36 ; 62, 64 ; 76, 78) des éléments de revers (26, 28) s'étendant des deux côtés se terminent dans la zone avant (6) et/ou dans la zone arrière (8) à distance les unes des autres sur l'extrémité longitudinale (40).

9. Article d'hygiène selon une ou plusieurs des revendications précédentes, **caractérisé en ce que** la distance (42) entre les lignes de base de revers (34, 36 ; 62, 64 ; 76, 78), dans un segment longitudinal central de l'article d'hygiène et en-dehors de ladite zone partielle (48, 50, 66, 68), est comprise entre 130 et 170 mm, en particulier entre 140 et 165 mm.

10. Article d'hygiène selon une ou plusieurs des revendications précédentes, **caractérisé en ce que** la distance (42) entre les lignes de base de revers (34, 36 ; 62, 64 ; 76, 78) est comprise, dans ladite zone partielle (48, 50, 66, 68), entre 160 et 220 mm, en particulier entre 180 et 200 mm, en particulier entre 185 et 195 mm.

11. Article d'hygiène selon une ou plusieurs des revendications précédentes, **caractérisé en ce que** la distance (42) entre les lignes de base de revers (34, 36 ; 62, 64 ; 76, 78), dans une zone d'extrémité du produit incluant une extrémité longitudinale de l'article d'hygiène, est comprise entre 100 et 180 mm, en particulier entre 100 et 160 mm, en particulier entre 100 et 150 mm, en particulier entre 100 et 140 mm, en particulier entre 100 et 130 mm, en particulier entre 105 et 120 mm.

12. Article d'hygiène selon une ou plusieurs des revendications précédentes, **caractérisé en ce que** le poids par unité de surface de la couche à corps absorbant (24, 60, 70) ou de l'un de ses matériaux absorbants augmente dans le sens transversal (44) ou dans le sens longitudinal (38) de 30 à 200 %, en particulier de 30 à 150 %, en particulier de 50 à 120 %.

13. Article d'hygiène selon une ou plusieurs des revendications précédentes, **caractérisé en ce que** le poids par unité de surface d'un matériau absorbant de la couche à corps absorbant (24, 60, 70) à l'extérieur de ladite zone partielle (48, 50, 66, 68) est compris entre 200 et 500 g/m², en particulier entre 250 et 450 g/m², en particulier entre 280 et 350 g/m².

14. Article d'hygiène selon une ou plusieurs des revendications précédentes, **caractérisé en ce que** le poids par unité de surface d'un matériau absorbant de la couche à corps absorbant (24, 60, 70) à l'intérieur de ladite zone partielle (48, 50, 66, 68) est compris entre 250 et 1000 g/m², en particulier entre 350 et 750 g/m², en particulier entre 500 et 700 g/m².

15. Article d'hygiène selon une ou plusieurs des revendications précédentes, **caractérisé en ce que** la distance d'une extrémité distale des éléments de revers (26, 28) verticaux par rapport à la ligne de base de revers (34, 36 ; 62, 64 ; 76, 78) à l'intérieur de ladite zone partielle (48, 50, 66, 68) est comprise entre 40 et 60 mm.

16. Article d'hygiène selon l'une des revendications précédentes, **caractérisé en ce que** la distance d'une extrémité distale des éléments de revers (26, 28) verticaux par rapport à la ligne de base de revers (34, 36 ; 62, 64 ; 76, 78) à l'extérieur de ladite zone partielle (48, 50, 66, 68) est comprise entre 30 et 40 mm.
